# EUROPEAN PATENT APPLICATION

(11) **EP 0 618 203 A1**
(43) Date of publication of application: **05.10.1994**
(21) Application number: 94104887.8
(22) Date of filing: 28.03.1994
(51) Int. Cl.: C07D 311/62, C12P 17/06, A61K 31/35

(54) **3-O-acylated catechins and method of producing same**

(30) Priority: 29.03.1993 JP 91934/93
(71) Applicant: MITSUI NORIN CO., LTD., Tokyo (JP)
(72) Inventor: Sakai, Miwa c/oFood Research Lab., Fujieda-shi Shizuoka-ken (JP); Suzuki, Masayuki c/oFood Research Lab., Fujieda-shi Shizuoka-ken (JP); Nanjo, Fumio c/oFood Research Lab., Fujieda-shi Shizuoka-ken (JP); Hara, Yukihiko c/oFood Research Lab., Fujieda-shi Shizuoka-ken (JP)
(74) Representative: VOSSIUS & PARTNER

(57) **Abstract**

The present invention discloses novel acylated compounds of formula (I) and an efficient method of producing same from free catechins. Moreover, since these acylated catechin compounds have a much more superior solubility in fats and oils than any catechins previously known, they may be used as a highly effective antioxidative agents, 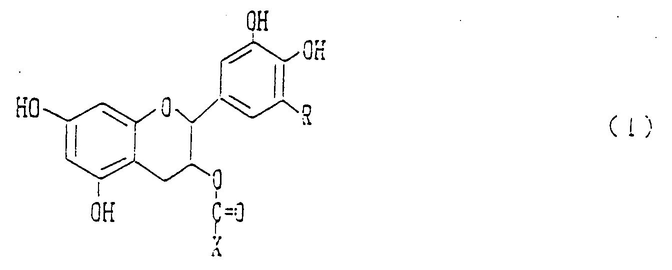
wherein X is an alkyl group of 1-10 carbon atoms, and R is a hydrogen atom or a hydroxyl group.

## Description

The present invention relates to 3-0-acylated catechin, the method of producing same and the use of same as an antioxidant.

Catechins are a type of polyphenol found in the roots, bark, seeds and leaves of various plants, in particular they are found in tea leaves (10-20% on a dried tea basis). Recently catechins have been discovered to have anti-oxidative, anti-bacterial, anti-viral, anti-mutagenic, anti-tumor properties, and to prevent a rise in blood glucose and cholesterol levels and blood pressure.

Out of all of these physiological properties, in particular the anti-oxidative property of tea polyphenols is strong, being not only superior to vitamin E which has been used up until now, but also having a superior anti-photo-oxidative effect on fats and oils. Thus it is expected that tea catechins may be used as a natural anti-oxidant.

However, one problem is that catechins have a low oil-solubility and so do not easily dissolve in fats and oils.

The present invention relates to a novel 3-0-acylated catechin with superior oil solubility, and an effective, efficient method of producing this compound. The present invention relates also to the use of the catechin as an antioxidative agent for lipids etc., and with the problem of low oil-solubility as mentioned above being resolved, it is expected that more superior antioxidative agent for fats and oils than has been available up until now, can be developed.

Therefore, an object of the present invention is to find a solution to the problem of low oil-solubility as mentioned above. This object has been achieved by the finding that by using the method of transesterification by carboxylesterase, a catechin acyl derivative with superior lipophilicity could be obtained expediently and efficiently.

The present invention relates to a 3-0-acylated catechin compound, which is represented by structural formula 1, 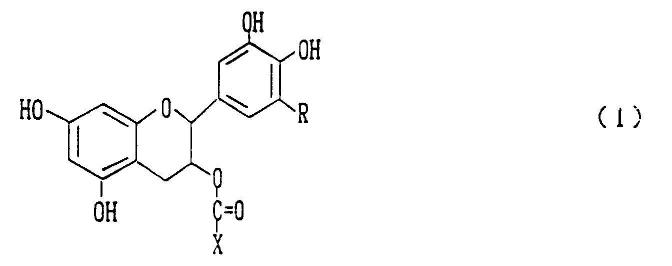
wherein X is an alkyl group of 1-10 carbon atoms, and R is a hydrogen atom or a hydroxyl group. The present invention furthermore relates to a process for the production of 3-O-acylated catechin compounds of above formula 1 which is characterized by the carboxylesterase action on free catechins as shown in formula 2, 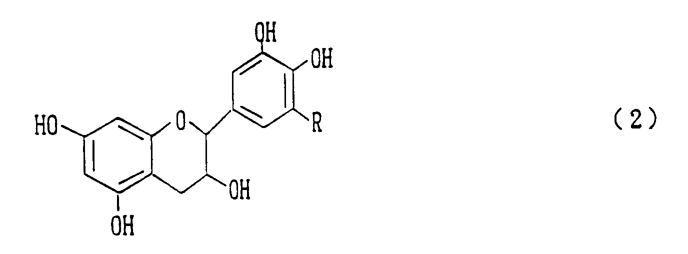
wherein R is a hydrogen atom or a hydroxyl group, and the carboxylesterase action on ester compounds as shown in formula 3, 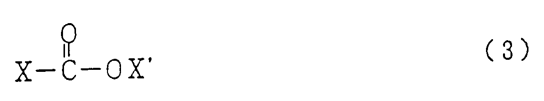
wherein X is an alkyl group of 1-10 carbon atoms, and X' is an aromatic group, glyceryl, acylglyceryl or an alkyl group of 1-10 carbon atoms. Preferably the aromatic group has 6-10 carbon atoms which can optionally be substituted, for example by a nitro group. Finally, the present invention relates to the use of this 3-O-acylated catechin as represented by formula 1, wherein X is an alkyl group of 1-10 carbon atoms, and R is a hydrogen atom or a hydroxyl group as an antioxidant.

Structures of the free catechins of the present invention are shown in above formula 2 and examples thereof include (-)-catechin, (-)-epicatechin. (-)-gallocatechin, (-)-epigallocatechin. These substances can be extracted and separated using the method described in US-A-4,613,672.

The structure of the ester compounds of the present invention is shown in formula 3, and can represent any ester which can be used as a carboxylesterase substrate. For example, methyl acetate. ethyl acetate, propyl acetate, butyl acetate, pentyl acetate, hexyl acetate, heptyl acetate, octyl acetate, methyl propionate, ethyl propionate, propyl propionate, butyl propionate, pentyl propionate. hexyl propionate, heptyl propionate, octyl propionate, methyl butyrate, ethyl butyrate, propyl butyrate, butyl butyrate, pentyl butyrate, hexyl butyrate, heptyl butyrate, octyl butyrate, methyl valerate, ethyl valerate, propyl valerate, butyl valerate, pentyl valerate, hexyl valerate, heptyl valerate, octyl valerate, methyl hexanoate, ethyl hexanoate, propyl hexanoate, butyl hexanoate, pentyl hexanoate. hexyl hexanoate, heptyl hexanoate, octyl hexanoate, methyl heptanoate , ethyl heptanoate, propyl heptanoate, butyl heptanoate, pentyl heptanoate, hexyl heptanoate, heptyl heptanoate, octyl heptanoate, metyl octanoate, ethyl octanoate, propyl octanoate, butyl octanoate, pentyl octanoate, hexyl octanoate, heptyl octanoate, octyl octanoate, phenyl acetate, phenyl propionate, phenyl butyrate, phenyl valerate, phenyl hexanoate, phenyl heptanoate, phenyl octanoate, p-nitrophenyl acetate, p-nitrophenyl propionate, p-nitrophenyl butyrate, p-nitrophenyl valerate, p-nitrophenyl hexanoate, p-nitrophenyl heptanoate, p-nitrophenyl octanoate, monacetin, triacetin, tripropionin, tributyrin etc. may be used.

The source of carboxylesterase need not be limited but if for example it is obtained from microorganisms, carboxylesterase derived from Streptomyces rochei, or Aspergillus niger is particularly desirable. The carboxylesterase produced from these microorganisms may be in a living or dried form, or it may be derived from any part thereof; alternatively it may be derived from either the crude enzyme or the purified enzyme. It may also be used in the form of an immobilized enzyme.

The acylated catechin compound as shown in fomula 1 could be for example an acetyl, propionyl, butyryl, valeryl, hexanoyl, heptanoyl, or octanoyl catechin compound as mentioned above.

To produce the acylated catechin compound of the present invention, an enzyme solution is added to a mixture of for example, ethyl acetate, which acts as the acyl donor, and free catechin, which acts as the acyl acceptor, to initiate the ester transfer reaction. When the acyl donor is a solid and does not easily dissolve in water, it can be dissolved in a suitable organic solvent (such as di-ethyl ether or acetonitrile) and added to the reaction. Other supplements, such as a buffer to control the pH of the reaction, may be added as necessary.

The molar ratio, and the amount of carboxylesterase or solvent used needs not to be strictly determined. However usually for every 1 mole of free catechins, 1-10 moles more of the ester compound should be used. The amount of carboxylesterase used should be about 10-100,000 units for every gram of free catechins, preferably between 100-5,000 units. The temperature and duration of the reaction is not strictly set, but should be between 20-60°C for between 1-100 hours. The pH of the reaction should be between pH 4-7.

The acylated catechin compound as described above, can be recovered from the reaction by for example using organic solvents, silica gel chromatography, high performance liquid chromatography etc.

The present invention relates to an antioxidative agent containing catechins acylated by carboxylesterase as the active ingredient, that is to say 3-0-acylated catechin as show in formula 1. The acylated catechin derivative may be added directly, or alternatively be dissolved in an organic solvent or an organic solvent solution before being added to fats and oils, foods which contain fats and oils, or the raw material thereof. A surfactant may be used to ensure it dissolves quickly in the fats and oils.

In order to demonstrate its antioxidative effect, 3-0-acylated catechin was added to fats and oils in a concentration of 0.01-0.1% by weight, and a safe, effective antioxidative action was observed. The antioxidative agent of the present invention may be used in conjunction with other antioxidative compounds which are in common use, for example α-tocopherol, propyl gallate, TBHQ, gallic acid, ascorbic acid, organic acid, tert-butyl-hydroxy anisol(BHA), or 3-5-Di-tert-butyl-4-hydroxy toluene(BHT).

### Examples

The present invention is explained in more detail in the following examples.

### Example 1

800 units of carboxylesterase derived from Streptomyces (product of Wako), 10ml of Macllvaine buffer (pH5.0), and 200mg of (±)-catechin (product of Sigma) were mixed into 20ml of ethyl acetate.

The above solution was shaken for 24 hours at a constant temperature of 33°C. After this time the reaction solution was evaporated and injected into an ODS column (6×45cm). A 40% aqueous methanol solution (containing 0.01% trifluoroacetic acid) was used as an eluant solvent. The fraction containing the desired substances was collected and after removal of methanol by evaporation, the solution was subjected to adsorption in a polyamide gel column. The column was washed with distilled water and then the product was eluted with 90% aqueous methanol. This solution was evaporated again and freeze dried, yielding 90mg of (-)-3-0-acetyl catechin, the structure of which was determined by MS and NMR anaylsis. The date is shown below.
FAB-MS(positive): m/e 333(M+H)⁺ molecular weight 332
¹H-NMR(270MH_{z}, CD₃OD)
1.94(s,3H,acetyl-(CH₃), 2. 61(dd,H,H-4b), 2.74(dd,H,H-4a), 4.91(d,H,H-2), 5. 19(m,H,H-3), 5.88(d,H,H-8), 5.93(d,H,H-6), 6.66(dd,H,H-6' ), 6.73(d,H,H-5' ), 6.77(d,H,H-2' )

### Example 2

The ester transfer reaction was conducted using ethyl propionate instead of ethyl acetate to produce a propionyl compound of (-)-catechin. The conditions of the reaction were the same as in Example 1. The purified substance was passed through an ODS column and using 45% methanol (containing 0.01% triofluroacetic acid). Extraction was conducted in the same way as in Example 1, yielding 40mg of propionyl catechin. The structure of this (-)-3-0-propionyl catechin was determined by MS and NMR analysis. The date is shown below.
FAB-MS(positive):m/e 347 (M+H)⁺ molecular weight 346
¹H-NMR(270MH_{z}, CD₃OD)
0.99(t,3H,propyl-(CH₃), 2.22(m,2H,propyl-CH₂),2.60(dd,H,H-4b), 2.77(dd,H,H-4a), 4.83(d,H,H-2), 5.19(m,H,H-3), 5. 88(d,H,H-8), 5.93(d,H,H-6), 6. 67(dd,H,H-6' ) 6.73(d,H,H,-5' ), 6.78(d,H,H-2' )

### Example 3

This experiment was conducted using a crude enzyme derived from Aspergillus niger (product of Amano Seiyaku), to produce a propionyl compound of (-)-epigallocatechin. 200mg of (-)-epigallocatechin, 10ml of phenyl propionate, 10,000 units of carboxylesterase, 20ml of Macllvaine buffer (pH5.0) were mixed together and shaken at 33°C for 24 hours. The product formed was extracted with ethyl acetate and after evaporation it was sepateted and purified by silica gel chromatography.

An eluate of n-hexane, ethyl acetate and trifluoroacetic acid (in a ratio of 6:5:0.01 respectively) was used. The reaction yielded 110mg of propionyl epigallocatechin. The structure of this (-)-3-0-propionyl epigallocatechin was determined by MS and NMR analysis.
FAB-MS(positive):m/e 363 (M+H)⁺ molecular weight 362
¹H-NMR(270MHz, CD₃OD)
0.966(t,3H,propyl-CH₃-4), 2. 23(q,H,propyl-CH₂),2.77(dd,H,H-4b), 2.89(dd,H,H-4a), 4. 87(s,H,H-2), 5. 34(m,H,H-3), 5. 89(d,H,H-8), 5.93(d,H,H-6), 6. 45(s,2H,H-2',6' )

### Example 4

Phenyl butyrate was used instead of phenyl propionate to produce a butyryl derivative of (-)-epigallocatechin. Conditions of the reaction and method of sparation were the same as in Experiment 3. The reaction yielded 25mg of butyryl-epigallocatechin. The structure of this (-)-3-0-butyryl-epigallocatechin was determined by analysis. Data are shown below.
FAB-MS(positive):m/e 377 (M+H)⁺ molecular weight 376
¹H-NMR(270MHz, CD₃OD)
0. 761(t,3H,butyl-CH₃-4), 1.472(m,2H, butyl-CH₂-3),2.81(t,2H,butyl-CH₂-2), 2. 77(dd,H,H-4b), 2. 90(dd,H,H-4a), 4. 88(s,H,H-2), 5. 34(m,H,H-3), 5.89(d,H,H-6), 6. 46(s,2H,H-2',6' )

### Example 5

The solubility of (-)-3-0-acetyl catechin, (-)-3-0-propionyl catechin, (-)-3-0-propionyl epigallocatechin, and (-)-3-0-butyryl catechin in fats and oils was tested in the following way. Each sample was added to salad oil (Honen Corporation) in a concentration of 100ppm, and turbidity was determined by measuring absorbance at 660nm.

**Table 1**

| Compound | OD 660 |
|---|---|
| Control | 0.057 |
| (-)-Catechin | 0.162 |
| (-)-Epigallocatechin | 0.277 |
| (-)-3-0-Acetyl catechin | 0.055 |
| (-)-3-0-Propionyl catechin | 0.056 |
| (-)-3-0-Propionyl epigallocatechin | 0.070 |
| (-)-3-0-Butyryl epigallocatechin | 0.059 |

As shown in Table 1, it was confirmed that the acylation of (-)-catechin and (-)-epigallocatechin increased their respective solubilities in fats and oils.

### Example 6

We investigated the antioxidative activity of the acylated derivatives of (-)-epigallocatechin, one of the most powerful antioxidants in tea. (-)-Epigallocatechin, (-)-3-0-propionyl epigallocatechin and (-)-3-0-butyryl epigallocatechin were dissolved in salad oil (product of Honen Corporation), in a concentration of 100ppm respectively, and using the Rancimat method, the induction time was used as a measure of the level of antioxidativity. (The reaction was conducted at a temperature of 120°C). Results are shown in Table 2.

No differences were observed between the induction times of (-)-epigallocatechin and its derivitives, showing that acylation does not cause a decline in antioxidative activity.

**Table 2**

| Compound | Induction Time (hr) |
|---|---|
| Control | 3.40 |
| (-)-Epigallocatechin | 6.03 |
| (-)-3-0-Propionyl epigallocatechin | 6.02 |
| (-)-3-0-Butyryl epigallocatechin | 6.07 |

## Claims

1. A 3-0-acylated catechin compound expressed by formula 1, 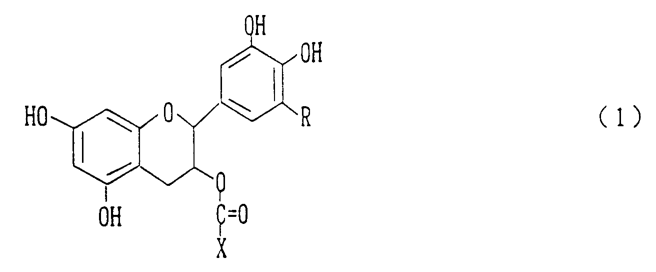 where X is an alkyl group of 1-10 carbon atoms, and R is a hydrogen atom or hydroxyl group.

2. A method of producing 3-0-acylated catechin according to Claim 1, which comprises, in the presence of free catechin as expressed by formula 2. 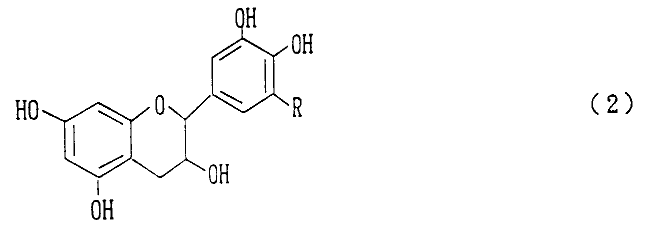 where R is a hydrogen atom or a hydroxyl group, subjecting carboxylesterase action on a ester compound as shown in formula 3, 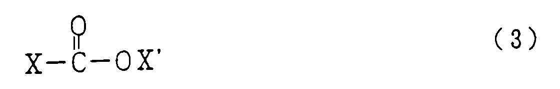 where X is an alkyl group of 1-10 carbon atoms, and X' is an aromatic group, glyceryl, acylglyceryl or an alkyl group of 1-10 carbon atoms.

3. The method of producing 3-0-acylated catechin according to Claim 2, wherein the free catechin is one selected from the group consisting of (-)-catechin, (-)-epicatechin, (-)-gallocatechin and (-)-epigallocatechin.

4. The method of producing 3-0-acylated catechin according to Claim 2 or 3, wherein the amount of the ester compound used is 1-10 moles per 1 mole of the free catechin.

5. The method of producing 3-0-acylated catechin according to any one of Claims 2 to 4, wherein the enzymatic reaction is carried out under conditions of pH 4-7 at a temperature of 20-60°C for 1-100 hours.

6. An antioxidative agent containing 3-0-acylated catechin according to Claim 1 as an effective component.

7. The antioxidative agent as claimed in Claim 6 wherein the amount of 3-0-acylated catechin is in a range of 0.01-0.1% by weight based on the fats and oils.
